# EUROPEAN PATENT APPLICATION

(11) **EP 3 878 491 A1**
(43) Date of publication of application: **15.09.2021**
(21) Application number: 20020118.4
(22) Date of filing: 10.03.2020
(51) Int. Cl.: A61M 5/00

(54) **MEDICAL DEVICE PACKAGING WITH NFC PAIRING SUPPORT**

(71) Applicant: Ypsomed AG, 3401 Burgdorf (CH)
(72) Inventor: Labudde, Marc, CH-3012 Bern (CH); Kühni, Florian, CH-3007 Bern (CH)

(57) **Abstract**

The present invention describes a packaging item adapted for transport or storage of a component used to conduct a medical therapy, where the packaging item includes a guiding element to support a user in establishing data communication between a medical device for administering a drug to a patient and a remote device for monitoring or controlling the medical therapy; a medical therapy system where establishing data communication is supported by a packaging item; and a method for establishing data communication in a medical therapy system using a guiding element included in a packaging item used for transport or storage one of the system components.

## Description

### FIELD OF THE INVENTION

The present invention relates to a system for conducting a medical therapy, comprising a medical device for delivering a drug to a patient or measuring a therapy relevant parameter from a patient, a remote device for monitoring or controlling the therapy, and a packaging item for transport or storage of a system component.

### BACKGROUND OF THE INVENTION

In the application of drug administration devices or measurement devices for physiological parameters it is of great importance that the user is interacting with the device he or she is actually intending to use. For example, a patient or healthcare professional HCP administering a drug needs to be sure that the command to inject a certain amount of drug is executed by the intended device on the patient's body. While evident in the case of entirely mechanical devices, this can be difficult to ensure when the medical device is part of a distributed therapy system, where components may not be in contact or even nowhere near the patient's body. Examples of such systems are a drug delivery system with a remote control unit or smartphone for controlling the drug delivery device, a diabetes management system with a glucose sensor and a smartphone for collecting and processing data, or a combination of the two systems. In a more complex therapy system, the medical device may also include a combination of a drug delivery device and an add-on releasably attachable to the delivery device for providing a communication interface. An other possible combination is a remote device built into a second medical device, such as a blood glucose measurement device equipped with an extended user interface and system control functions. In all these configurations a remote device is communicating wirelessly with the medical device and handling the exchange of data with other system components. By validating the link between the medical device and the remote device the user can ensure that the medical device addressed by the remote device is the same as the user intends to apply. This process, known as pairing function, has two aspects: to verify that the two devices can correctly exchange data on a technical level, and to make sure the communication is established between two devices the user is actually intending to use. Pairing is typically realised by asking the user to bring the two devices into physical contact or close proximity to each other. Such a contact or close proximity can be detected by a communication system, clearly distinguished from proximity to other devices of the same kind and hence used as proof of authenticity of a link between the two devices. The authentication protocol may be controlled by any component of the therapy system. As a result of the pairing function, a validated link is established between the medical device and the remote device, providing a reliable basis for the therapy system and the application of drug administration devices or measurement devices for physiological parameters.

One way to ensure correct pairing of the medical device and the remote device is to use a short range communication technology such as electric contacts, RFID or NFC. With such a link, any successful communication between the medical device and the remote device is proof of physical proximity. The maximum distance for successful pairing depends on the technology used. Typical values may be zero for electrical contacts, 1cm to 5cm for low power NFC, 10cm for standard NFC, up to a practical maximum of 50cm as a possible limit to avoid unintended pairing with a second medical device. Alternatively, the pairing can be achieved by using a wider range communication system and use an estimate of the distance between the medical device and the remote device to detect sufficient proximity. More complex communication systems may provide other indicators such as information about a relative direction, a relative position or an absolute position which can be used in the same way. In all these cases, an indication of sufficient proximity is used to validate the authenticity of the communication link.

In order to realise the communication between the medical device and the remote device, each of the devices has a communication unit which can receive and/or transmit data. The communication unit can for example be an antenna or an electric contact. Therefore, it is the distance between the two communication units which is relevant for pairing.

While close proximity is useful to ensure correct pairing of the medical device and the remote device, the user needs to know where exactly the communication unit is located on each of the devices. The pairing position can be difficult to find, especially for handheld or wearable medical selfcare devices such as blood glucose measurement devices, auto-injectors or injection pens which tend to be small and not suitable to incorporate obvious marking or positioning features in close vicinity of the communication unit. There clearly is a particular need for pairing support in this context.

The following documents disclose medical systems with a medical device and a remote device, where pairing potentially could be an issue:
EP 3539593 A1 describes a drug delivery system where a drug administration device is manually inserted in a docking station to communicate with other devices.
WO2017/059553 A1 describes a drug delivery system where a drug administration device uses NFC to read data from a packaging item.
US2018182491 describes a drug delivery system where a glucose measurement device is monitored by a smartphone using NFC communication, and where the pairing function to ensure authentication of the connection between the smartphone and the glucose sensor is supported by visual, haptic or acoustic feedback generated by one of the devices.
WO2016/009202 describes a packaging for a delivery device for vaporised substances where the packaging is actively involved in the implementation of a number of features using NFC communication.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to a direction or orientation towards the end of the drug delivery device carrying an injection needle or an injection cannula, whereas the term "proximal" is meant to refer to the opposite direction or orientation towards the end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to support the authentication of a communication link between a medical device and a remote device by means of a packaging item. The packaging item could for example be disposable or for multiple use, for transport or storage, in one piece or an assembly of multiple parts, a box with an information leaflet, a blister packaging with a lid or a bag to carry the medical device together with accessories. The medical device can for example be a drug delivery device for subcutaneous or intramuscular administering of a liquid medication to the patient, a drug delivery device with an add-on accessory attachable to said delivery device, or a device for measuring a physiological parameter. The remote device can for example be a smart phone, tablet computer, a dedicated remote control device or a communication device for forwarding data from the medical device to an other system component or vice versa. Alternatively, the remote device can be a further medical device which acts as remote device when it comes to controlling or monitoring the therapy. As part of the implementation of the electronic communication between the remote device and the medical device, a communication unit is integrated in each of the devices, which is configured to transmit and/or receive data. In case of a wireless communication technology this will typically be an antenna, in other cases an arrangement of one or more electrical contacts. As the authentication of the communication link is based on physical contact or close proximity, the exact relative position and orientation of the communication units is relevant for the success of the pairing.

The packaging item can either be used for storage or transport of one of the devices, of a component thereof, of an accessory, of a consumable or of any other component involved in conducting the therapy.

The objective of the invention is achieved by integrating a guiding element into the packaging item which supports the user in bringing the medical device and the remote device into a relative position and/or orientation for transmitting and/or receiving data.

The guiding element on the packaging item indicates to the user a preferred relative position and/or orientation of the two devices. The indication can depict one of the devices itself, both devices, a communication unit, a part of or the complete silhouette, or any other characteristic feature of one or both devices. Alternatively, the indication can mark the position of a communication unit. In case of a visual indication the user can easily recognise how to position the two devices for pairing, without having to consult a manual or remembering a training. In case of a haptic indication the user is even haptically guided to put the devices into pairing position, making the positioning more precise and quicker.

With a packaging item according to the present invention, easy, quick and reliable pairing of the remote device with the medical device is possible. A new kind of medical therapy system can be formed, where the packaging of any system component can be used to ensure easy, quick and reliable pairing of a remote device with a medical device and hence improve ease of use, reliability and safety of the therapy.

This is even more advantageous for users with impaired visual or tactile senses as often the case with patients requiring medication.

To monitor or control a therapy in a system according to the present invention, a remote device is typically paired with a medical device by conducting the following steps:
- using the guiding elements on the packaging of a system component, the user brings the remote device and the medical device to a relative position and/or orientation for exchanging data;
- the system, typically the remote device, establishes a communication link between the remote device and the medical device;
- the system, typically the remote device, determines whether the two devices are closer than the maximum distance allowed for pairing. Such a maximum distance refers to the distance between the communication unit in the medical device and the communication unit in the remote device and will typically be specified in the range of zero to 50cm. For optimal communication and most reliable pairing, the maximum distance will be as small as possible, preferably not exceeding 5cm.
- the system, typically the remote device, performs a pairing function by validating the communication link between the remote device and the medical device if the maximum inter-unit distance is smaller than or equal to the specified maximum.

While the control of communication and of the pairing process is typically performed by the remote device, it is obvious that any device in the therapy system could perform this task in communication with the remote device, including the medical device, or the task could be distributed over more than one devices. The same is true for the user interface for monitoring or controlling the therapy, which could be implemented on the remote device, on any other device in communication with the remote device, the medical device, or on any combination of devices in the therapy system.

Turning now to more specific details of the packaging item, in case of a visual guiding element it could comprise a printed mark or symbol. As described before, this element may indicate the position and/or orientation of either one of the devices, or either one of the communication units, or any combination thereof. In case of a haptic guiding element it could comprise a recess or opening suitable for holding the medical device and/or the remote device. Alternatively, the haptic guiding element could comprise a mechanical structure such as an indentation, embossment, rise or ridge.

A number of options is open to implement the pairing based on close proximity. An obvious way is to use a communication technology based on physical contact of the communication units, such as plain electrical contacts, or a wireless communication technology which is limited to a short range, preferably not exceeding 5cm. Such a communication only works in close proximity; hence any successful communication is proof of close proximity. Examples of such close range technologies, apart from electrical contacts, are Near Field Communication NFC, RFID technology and projected capacitive touch PCAP. Visual or infra-red optical communication technologies can also be adapted to have a restricted range. Alternatively, a wireless communication technology may be used with a range of more than 5cm, if proximity is detected by some other means. Examples of such communication technologies are Bluetooth®, ZigBee, Thread, Ant, GSM, UMTS or LTE. As most wireless communication technologies use indicators of signal strength for internal purposes, a pairing could for example use such a signal strength indication as an estimate of distance between communication partners, in particular as an estimate of distance between the communication units in the remote device and in the medical device. The pairing control could then include a comparison of the distance estimation with the specified maximum inter-unit distance and otherwise work just as described for near field technologies. Other features of the communication technology, such as an indication of the spatial direction or an indication of the absolute or relative position could be used in a similar way or combined with distance estimation to obtain proof of proximity and validate the communication link.

Once the remote device is successfully paired with the medical device, the communication link between the two devices can safely be used to monitor or control the therapy. This will involve data being exchanged over the communication link in both directions. The remote device may read, store, process or display therapy relevant data from the medical device or forward the data to an other device of the therapy system. The medical device may receive, store, process or display therapy relevant data from the remote device, for example to initiate the administration of medication.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a first preferred embodiment with a patch injector, a smartphone and a visual guiding element for pairing by NFC, printed on a patch injector packaging
- Fig. 2: depicts a second preferred embodiment with a patch injector, a smartphone and a visual guiding element for pairing by NFC, printed on a Quick Reference Guide shipped as part of a packaging
- Fig. 3 and Fig. 4: depict two further preferred embodiments with an add-on for an auto-injector, a smartphone and a haptic guiding element for pairing by NFC, integrated in a packaging for an add-on
- Fig. 5: depicts a further preferred embodiment with a BG measurement device, a smart watch and a haptic guiding element for pairing by Bluetooth®, integrated in an accessory bag for a glucose measurement day kit

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

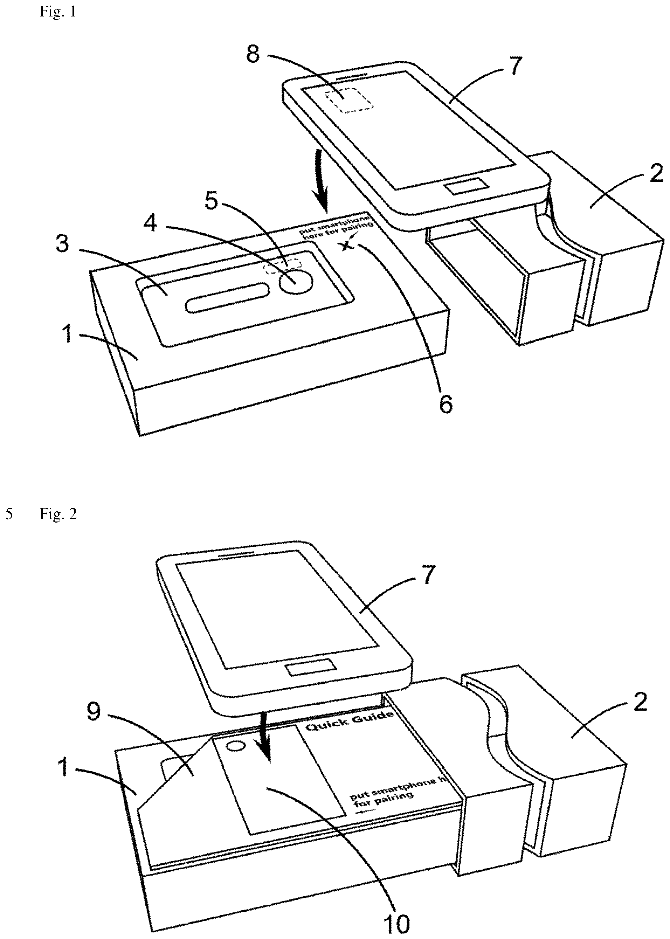

The present invention generally provides a packaging item adapted to support a pairing function of devices in a medical therapy system. While this could be a system for conducting any kind of medical therapy, those including active participation of the patient will benefit most of the pairing support, because patients will typically be less proficient in handling medical devices than healthcare professionals HCP, especially when suffering from conditions limiting the visual faculty or fine motor skills. Preferred therapies hence include those involving self-administering a liquid drug, such as Insulin, and/or self-measuring a physiological parameter, such as blood glucose level. Consequently, a first preferred embodiment of the invention may comprise a packaging item in a therapy system that includes delivering a drug and controlling the drug delivery using a smartphone. Any packaging in the therapy system could be adapted to provide support for the pairing of the drug delivery device with the smartphone. The packaging of reusable components, however, may be lost or disposed of at the time of application of the medical device. It may therefore be advantageous to adapt the packaging of a disposable or consumable component of the therapy system to include the guiding element. **Fig. 1** shows an example of such a packaging item, where a disposable patch injector (3) is shipped in a box (1) with a lid (2). The box, when closed, may allow safe transport and/or storage of the patch injector. In this example, the user opens the lid (2) of the packaging to gain access to the injector and activates the delivery device by pressing an activation button (4) on the top of the device. For the purpose of pairing the way of activating the patch injector is of no importance; the activation may also be automatic when opening the box, may be initiated by any other trigger, or the medical device may even be shipped in a state of permanent activation. When activated, the patch injector uses the communication unit (5) to wait for a remote device to establish a connection. Again, the scope of the invention is not limited to a particular way of establishing communication; in the example described here the patch injector may use a standard Near Field Communication NFC technology with a corresponding NFC antenna as communication unit and use a NFC communication protocol. The therapy system may further comprise a software for controlling and monitoring the therapy, configured to run on a smartphone (7) and to use the same communication technology as the medical device. Further, the smartphone may comprise a communication unit, in this example an NFC antenna (8). The patient may now activate the therapy control software on the smartphone. The smartphone may start establishing a communication link with the patch injector using the NFC protocol. In most cases this will not immediately be successful because the distance between the communication units is greater than the maximum range of the NFC technology as achievable in the given circumstances. In the embodiment described here the box has a printed mark (6) to indicate the position of the communication unit (5) in the patch injector (3). By means of the visual guidance provided by the printed mark (6) on the box (1) the patient may easily bring the smartphone to a position where the communication units (5) and (8) are sufficiently close so that the therapy control software can successfully establish a NFC communication link and perform the pairing function. Once paired, the smartphone may use any kind of communication to interact with the patient and other devices to control and/or monitor the medical therapy.

In a second preferred embodiment, illustrated in **Fig. 2****,** the guiding element could be integrated in a different component of the packaging item, for example on a quick guide manual (9). In this example, the guiding element is a printed representation of the complete remote device (10) rather than the communication unit of the remote device.

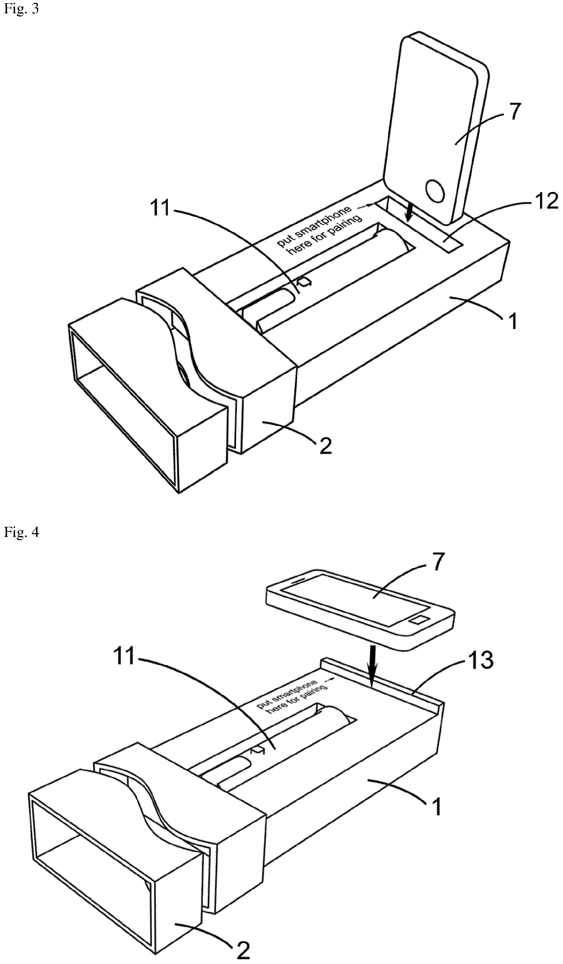

As an example including a haptic guiding element, a third preferred embodiment could provide an opening (12) in a packaging item, as shown in **Fig. 3****.** In this example the medical device is an injection device with an add-on (11). In a first step the patient operates the smartphone and the add-on in the packaging to initiate establishment of a communication link between the two devices just as described for the first preferred embodiment. In a second step the patient inserts the smartphone (7) into the opening (12) for pairing. After successful pairing the therapy system is ready to use the validated link. Activation, validation and use of the link can be conducted before and/or after drug delivery using the injection device.

One possible variation of the second preferred embodiment is shown in **Fig 4****.** Here, the guiding element is be a mechanical structure such as a ridge, in the present example the rim (13) of the packaging box (1).

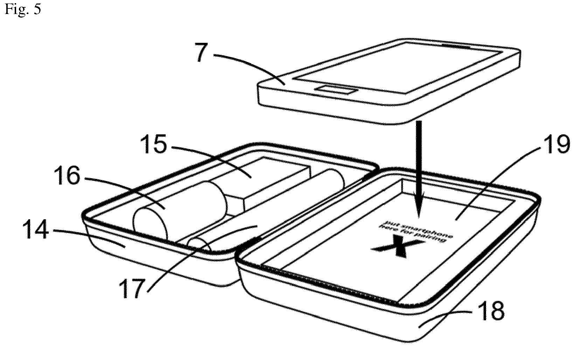

Another way of providing the pairing guidance is to include the guiding element into a packaging item adapted for multiple use, for example a carrying case for a medical therapy kit with all the components needed for a day. **Fig. 5** illustrates this third preferred embodiment with an example where the medical device is a glucose measurement device (15) carried in a case (14) together with measuring strips (16), a finger pricking device (17) and other equipment. The lid (18) of the carrying case forms a bay (19) to hold the smartphone during pairing. As the carrying case of this embodiment is larger than a typical disposable packaging, in this example the communication technology used is Bluetooth® and a combination of estimate of distance and estimate of direction of the communication link is used as an acceptance criterion for pairing.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Box
- 2: Lid
- 3: Patch injector
- 4: Activation button
- 5: Communication unit
- 6: Printed mark
- 7: Smartphone
- 8: Communication unit / NFC Antenna
- 9: Quick Reference Guide
- 10: Printed representation of a remote device
- 11: Add-on for an injection device
- 12: Opening
- 13: Rim / ridge
- 14: Carrying case
- 15: Glucose measurement device
- 16: Glucose measurement strips
- 17: Finger pricking device
- 18: Lid
- 19: Bay

## Claims

**1.** A packaging item adapted for transport or storage of a component used to conduct a medical therapy, the therapy involving:
a handheld or wearable medical device for administering a drug to a patient, or for measuring a parameter from a patient, having a first communication unit; and
a remote device for monitoring or controlling the medical therapy, having a second communication unit;
**characterised in that** the packaging item comprises a guiding element to support a user in bringing the first communication unit and the second communication unit into a relative position and/or orientation for transmitting and/or receiving data .

**2.** The packaging item according to claim 1, wherein the guiding element comprised in the packaging item comprises a visual indication, preferably a printed mark or symbol indicating
- a position and/or orientation of the first and/or second communication unit for pairing, and/or
- a position and/or orientation of the medical device and/or the remote device for pairing.

**3.** The packaging item according to claim 1, wherein the guiding element comprised in the packaging comprises a haptic indication of
- a position and/or orientation of the first and/or second communication unit for pairing, and/or
- a position and/or orientation of the medical device and/or the remote device for pairing.

**4.** The packaging item according to claim 3, wherein the guiding element comprised in the packaging is a recess or opening suitable for holding the medical device and/or the remote device in the position and/or orientation for pairing.

**5.** The packaging item according to claim 3, wherein the guiding element comprised in the packaging includes a mechanical structure such as an indentation, embossment, rise or ridge.

**6.** A medical therapy system, comprising:
a handheld or wearable medical device for administering a medication to a patient or measuring a therapy relevant parameter from a patient, comprising a first communication unit for transmitting and/or receiving data;
a remote device for monitoring or controlling the , comprising a second communication unit for receiving and/or transmitting the data; and
a packaging item adapted for transport or storage of a component of the therapy system, preferably the drug delivery device;
**characterised in that** the packaging item comprises a guiding element to support a user in bringing the first communication unit and the second communication unit into a relative position and/or orientation for transmitting and/or receiving the data, preferably for pairing.

**7.** The medical therapy system according to claim 6, wherein the medical device is a drug delivery device for administering the liquid medication to the patient, or a drug delivery device for administering the liquid medication to the patient with an accessory releasably attachable, or a device for measuring a blood glucose concentration.

**8.** The medical therapy system according to claims 6 or 7, wherein the first communication unit and the second communication unit interact with each other by means of a communication technology or protocol ensuring physical contact of the communication units or close proximity of said units with a maximum inter-unit distance ranging from zero to 50cm, preferably zero to 5cm.

**9.** The medical therapy system according to claim 8, wherein the first communication unit and the second communication unit interact with each other by means of one or more electric contacts, or by means of Near Field Communication NFC or RFID technology, or by means of optical or infrared communication, or by means of capacitive sensing such as projected capacitive touch PCAP.

**10.** The medical therapy system according to claim 8, wherein the first communication unit and the second communication unit interact with each other by means of a wireless communication technology with a maximum range of more than 5cm, and wherein an estimate of the relative position or a distance between the communication units is made and compared with the maximum inter-unit distance.

**11.** The medical therapy system according to claim 10, wherein the first communication unit and the second communication unit interact with each other by means of Bluetooth®, ZigBee, Thread, Ant, GSM, UMTS or LTE technology.

**12.** The medical therapy system according to claims 6 to 11, wherein the remote device is adapted to read, store, process or display therapy relevant data from the medical device or adapted to forward therapy relevant data to an other system component. The medical therapy system according to claims 6 to 11, wherein the medical device is adapted to receive, store, process or display therapy relevant data from the remote device, preferably to initiate the administration of medication.

**14.** The medical therapy system according to claims 12 or 13, wherein the remote device is a mobile electronic assistance device, preferably a smartphone, a tablet computer or a smart watch, or a further medical device.

**15.** A method for applying a handheld or wearable medical device for administering a medication to a patient or measuring a parameter from a patient in a therapy using a remote device and a packaging item, wherein
the medical device comprises a first communication unit for transmitting and/or receiving data, integrated in the medical device or as part of an accessory attachable to the medical device;
the remote device for monitoring or controlling the therapy comprises a second communication unit adapted for receiving and/or transmitting data;
the packaging item is adapted for transport or storage of a component required to conduct the therapy, preferably the drug delivery device or a part or accessory of the drug delivery device, and comprises a guiding element to support a user in bringing the first communication unit and the second communication unit into a relative position and/or orientation for transmitting and/or receiving the data; and
the method comprises the following steps:
bringing the remote device and the medical device to a relative position and/or orientation for receiving and/or transmitting data using the guiding element on the packaging item;
establishing a communication link between the medical device and the remote device;
determining, implicitly by successful communication or explicitly by using an estimate of the inter-unit distance between the first and the second communication unit, whether said inter-unit distance is smaller than or equal to a maximum inter-unit distance ranging from zero to 50cm, preferably zero to 5cm;
performing a pairing function by authenticating the communication link between the remote device and the medical device if the inter-unit distance between the first and the second communication unit is smaller than or equal to the maximum inter-unit distance; and
using the paired devices in the therapy.
